# EUROPEAN PATENT APPLICATION

(11) **EP 2 725 092 A1**
(43) Date of publication of application: **30.04.2014**
(21) Application number: 13461537.6
(22) Date of filing: 10.07.2013
(51) Int. Cl.: C12M 1/00, C12M 1/34

(54) **Device for breeding phototropic micro-organisms**

(30) Priority: 23.10.2012 PL 40133012 P
(71) Applicant: Instytut Agrofizyki im. Bohdana Dobrzanskiego PAN, 20-236 Lublin (PL)
(72) Inventor: Tys, Jerzy, 20-815 Lublin (PL); Gózdz, JAN, 20-810 Lublin (PL); Krzeminska, Izabela, 20-470 Lublin (PL); Wiacek, Dariusz, 20-850 Lublin (PL); Kwietniewska, Ewa, 20-492 Lublin (PL); Paul, Grzegorz, 20-601 Lublin (PL)
(74) Representative: Kalita, Lucjan

(57) **Abstract**

Apparatus comprising interconnected: a process water storage tank (1), a transition tank (2), an exchanger (3) formed of: a pasteurization, a recuperation and a cooling sections, an ice water aggregate (5), a heating water tank (6), a photobioreactor (7), a reproduction and heating pure cultures tank (8), a prepared nutrients heating tank (9), a carbon dioxide gas tank (10), a mixing pump (11), a centrifuge (12), and a biomass storage tank (13), creating a functional installation, characterized in that, the photobioreactor (7) in its spacious closed tank (14) has at least one light element (19) and this element is formed of a cylindrical transparent tube (20) of ring-shaped, circular cross section inside which a column (22) with LED light-emitting diodes (25) embedded on its walls is placed. The tube (20) is sealed from the bottom with a lid (21) and its top edges project above the photobioreactor's tank (14) closing cover (17). The column (22) is formed of a pass-through tube, and includes a fan placed on the top. On the outer side of the tube (20) and parallel to its axis and opposing to each other vertical collectors (26) powered by a mixing pump (11) are located, in these collectors hydraulic nozzles (27) are mounted, with it's outlets facing the outer surface of the tube (20).

Apparatus applies the light-emitting diodes LED (25) emitting light at wavelengths 450 - 470 nm - blue light, 650 - 670 nm - orange light and 700 - 740 nm - red light. The inner wall of the photobioreactor's tank (14) constitutes reflective surface (30) of the light emitted and the walls itself constitute a heating jacket (15) with the outer insulation layer (16).

Mixing nozzles (31) are mounted in the sidewall of the tank (14) equipped with a turbidity sensor (47).

## Description

Apparatus for cultivation of phototrophic microorganisms.

The invention relates to an apparatus for the cultivation of phototrophic microorganisms, in particular microalgae that show a high capacity of biomass production.

The cultivation of phototrophic microbes, such as micro algae, cyan bacteria and purple bacteria, takes place in photobioreactors, in which the growth and reproduction of these cells or intensification of the production of various substances is achievable by using phototrophic cells.

Process of biomass production from micro algae requires delivery of carbon dioxide, proper light levels and supply of other nutrients supporting the cultivation, while maintaining the lowest operational costs. For this purpose industrial scale installations with photobioreactors are constructed, in which, under the number of controlled parameters, biomass multiplying takes place.

Publication US4952511 discloses a photobioreactor in the form of a vertical container with internal vertical tubes made of transparent material. The tubes are equipped with illuminating lamps from the top and reflective mirrors from the bottom. The photobioreactor has a mixing pump, a system to maintaining constant temperature and a carbon dioxide supply connector.

Application US20110111490 presents a photobioreactor, which can use sunlight as the light source, yet to achieve wavelengths the most favorable for breeding, wavelength shifters described in the application are used.

Polish patent description PL191853 discloses a photobioreactor, in the volume of which, strong mixing together with high turbulence is performed, what causes uniform light distribution, gases exchange and temperature compensation. Furthermore the photobioreactor may have wavelength shifters installed.

From Japanese patent application JP2002315569 there is also known a method of the algae cultivation, that as a source of light uses LED diodes emitting monochromatic light of wavelength ranges 630 ÷ 690 nm and/or 400 ÷ 460 nm, what allows the production independent of the natural light sources.

The purpose of the invention is to provide such an apparatus for the industrial microalgae biomass cultivation, so the cultivation process takes place in a continuous and hermetic manner, and is independent of the external weather conditions.

The essence of the apparatus for the cultivation of phototropic microorganisms, especially microalgae, according to the invention lies in the fact that it includes interconnected: a process water storage tank, a transition tank, an exchanger in a pasteurization section, a recuperation section and a cooling section, an ice water generator, a heating water tank, a photobioreactor, a tank for reproduction and warming of pure cultures, a nutrients warming tank, a carbon dioxide gas tank, a mixing pump, a centrifuge, and a biomass tank, creating a functional system and it is **characterized in that**, the photobioreactor has at least one light element placed in its spacious closed tank and this element is formed of a cylindrical transparent, ring-shaped, with circular cross section tube and a column with LED light-emitting diodes embedded on its walls, that is placed inside the tube. The tube is sealed from the bottom with a lid and its top edges project above the photobioreactor's tank closing cover. The column is formed of a pass-through tube, where upper outlet is profiled and includes a fan, while on the outer side of the transparent tube and parallel to its vertical axis at least two vertical collectors powered by the mixing pump are located, in the collectors the hydraulic nozzles are mounted, with theirs outlets facing the outer surface of the tube. The jets of solution flowing under pressure from the nozzles placed on the collectors wash away and reduce the deposition of microorganisms on the outside of the tube.

Preferably, the apparatus incorporates light-emitting diodes LED emitting light at wavelengths 450 - 470 nm - blue light, 650 - 670 nm - orange light and 700 - 740 nm - red light. Incorporating the light waves of the indicated ranges enhances photosynthesis and chlorophyll absorption.

Preferably, the inner wall of the photobioreactor's tank includes reflective surface for the light emitted, what increases the effectiveness of exposure.

Preferably, the walls of the photobioreactor's tank constitute a heating jacket with the outer layer of insulation allowing maintenance of a constant temperature optimal for the cultivation.

Preferably, in order to avoid excessive deposition of microorganisms on the internal walls of the photobioreactor's tank and in order to regulate the illumination level of the cultivation, in its side wall, there are the mixing nozzles mounted, which constitute outlets of the conductors connected to the pump discharge connector. The mixing nozzles provide continuous turbulent mixing of the cultivation solution.

In the photobioreactor's tank a turbidity sensor preferably is located, which examines the extent of microalgae multiplying. At the appropriate microalgae concentration, using an automatic control, partial pumping out of the cultivation solution takes place and the photobioreactor is supplemented with process water. Implementation of the turbidity sensor allows to maintain the efficiency of the process on the effective level, which would drop significantly due to the shading of light caused by the continuous microorganisms multiplying.

The apparatus according to the invention allows, on the industrial scale, to utilize excessive amounts of carbon dioxide generated by industry, and efficiently obtain a significant amount of biomass. Used in the photobioreactor's tank the light element, formed of a cylindrical transparent tube projecting over the cover and placed inside the column tube equipped with a fan, allows ventilation of large number of the LED lights embedded in its walls. Application of efficient LEDs emitting light waves of different lengths allows for significant reduction in the unit cost of biomass produced. Equipment of the light element with hydraulic pressure nozzles, from which flowing out pressurized jets clean its outer surface, prevents from the formation of microalgae on the surface, so that the light from the LEDs easily penetrates the wall of the transparent tube. In addition, providing the inside surface of the tank wall with the reflective surface, causes that the light rays reflecting from the surface fully penetrate into the solution in the tank.

Similarly, it is preferable for the carried out in the photobioreactor process to use the heating jacket and mixing nozzles in the tank, as they ensure maintenance of a proper temperature and uniform composition throughout the volume.

A preferred embodiment according to the invention will be described with reference to the drawings, in which:

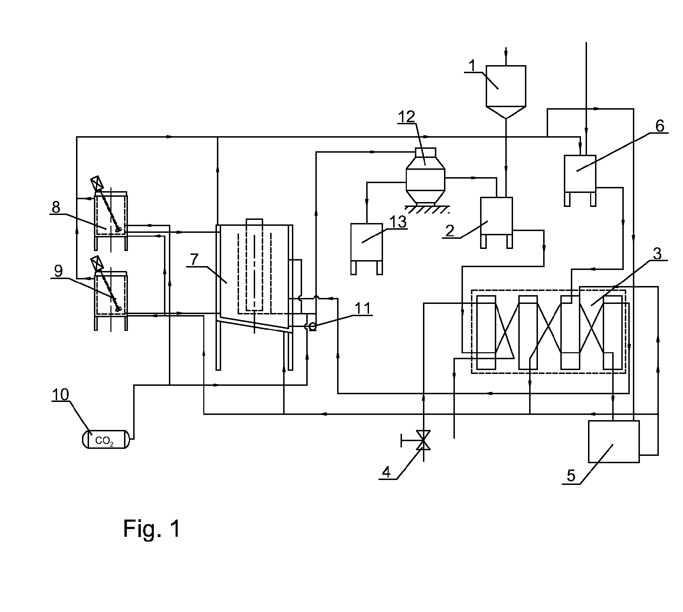

Fig. 1 shows the apparatus for the cultivation of phototrophic microorganisms schematically,

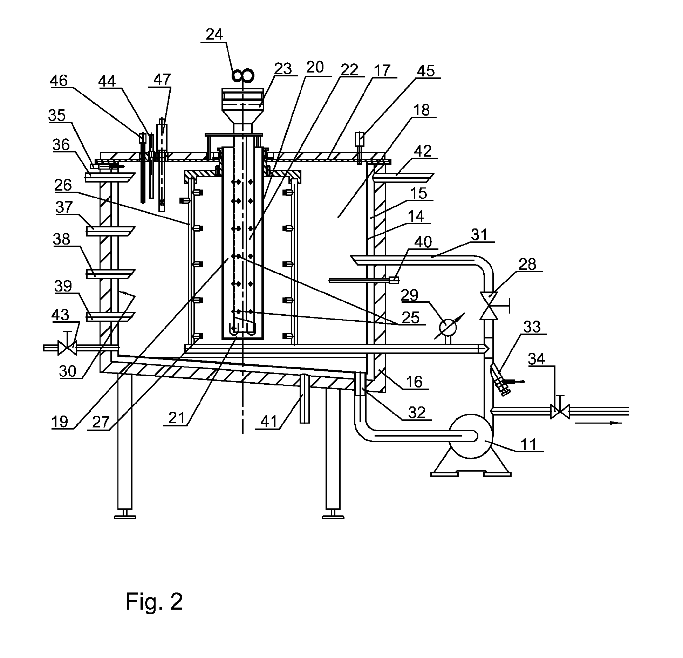

Fig. 2 shows a schematic longitudinal section of the photobioreactor, and

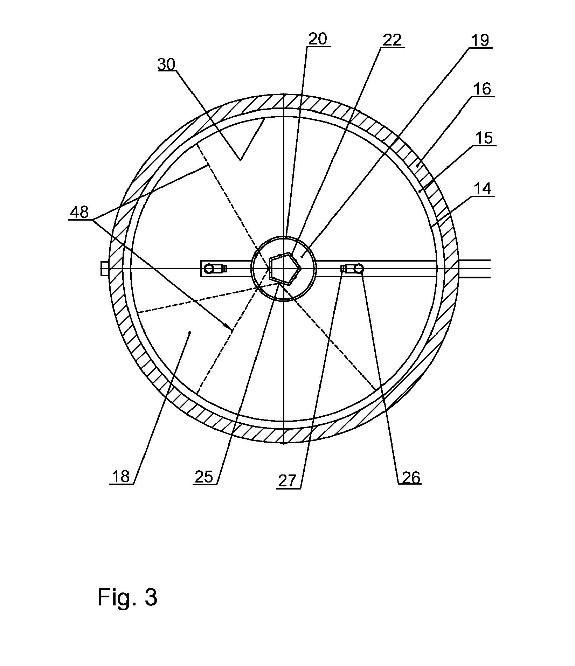

Fig. 3 shows a cross section through the photobioreactor`s tank.

In a preferred embodiment shown below, interconnection of elements forms a functional installation of the apparatus and includes both an explanation of the stages of the ongoing process and the function of these elements in this process.

In the apparatus according to the invention the process water required for the cultivation of microalgae is taken from a process water storage tank 1 of the and from a transition tank 2 and it is pasteurized in a pasteurization section of an exchanger 3, to which heating steam is fed by a control valve 4. In the recuperation section of the exchanger 3 portion of the heat is recovered during the partial cooling of the process water, and then, in the cooling section of the exchanger 3 the process water is cooled down to the optimal cultivation temperature. In order to finalize cooling of the process water an ice water from an aggregate 5 is used. Heating water from a tank 6 is heated in the recuperation section of the exchanger 3 to the optimal temperature for the cultivation and is directed to a photobioreactor 7 for diaphragmatic heating of the cultivation and to heat the contents of a reproduction pure cultures tank 8 as well as it is directed to a prepared nutrients heating tank 9. After filling the photobioreactor 7 with the process water, to a reaction chamber 18 strain of microalgae, other nutrients, and carbon dioxide from a stored gas tank 10, as a byproduct in the process of industrial production are dispensed.

The cultivation solution from the photobioreactor 7 is illuminated and subjected to turbulent mixing by a mixing pump 11, at a constant temperature. As a result of the signal from a turbidity sensor 47 built into the photobioreactor 7 a process of automatic pumping of the partial volume of the cultivation solution to a centrifuge 12 takes place. Centrifuged biomass is collected in a storage tank 13 and depending on the further method used it is passed for fermentation or drying. The effluent is directed into the transition tank 2 and through the pasteurization section of the exchanger 3 is returned to the photobioreactor.

The photobioreactor 7 of this apparatus is constructed of a vertical photobioreactor's tank 14 with a heating jacket 15 and the outer layer of a thermal insulation 16. The photobioreactor's tank 14 is equipped with a separable, airtight lid 17. In the reaction chamber 18 a light element 19 is placed, formed of a cylindrical glass tube 20 with a circular cross-section and placed inside this tube 20 a column 22 with embedded on its walls LED light-emitting diodes 25. The LED light-emitting diodes 25 are spaced on the column 22 relatively to each other horizontally and vertically in such a way that lines 48 defining a light cone of two adjacent diodes intersect within the tube 20. The implemented light-emitting diodes LED 25 emit light at the wavelengths 450 - 470 nm - blue light, 650 - 670 nm - orange light and 700 - 740 nm - red light. The tube 20 is sealed from the bottom with a lid 21 and its top edges project above the photobioreactor's tank 14 closing cover 17. The column 22 is formed of a pass-through metal tube with a cross section in the shape of an equilateral polygon, where an upper outlet 23 is profiled and includes a fan 24. Around the transparent tube 20 in the two opposite, vertical collectors 26 hydraulic pressure nozzles 27 are located from where flowing pressurized jets clean the outer surface of said tube 20. The collectors 26, in which nozzles 27 are mounted, are supplied with pressurized technological liquid by the mixing pump 11, and the pressure is regulated by a valve 28 and controlled using a pressure gauge 29.

The interior wall of the photobioreactor's tank 14 creates a reflecting surface 30 for the light emitted.

In the sidewall of the photobioreactor's tank 14 mixing nozzles 31 are mounted and they constitute outlets of the conductors connected to the mixing pump 11 discharge connector, and the pump is connected to the photobioreactor's tank 14 through a suction connector 32. In addition, the pump discharge connector is provided with a carbon dioxide dosing valve 33 and a valve 34, which feeds the cultivation solution for centrifugation. In addition, in the sidewall of the photobioreactor's tank 14 there is a water level sensor 35 protecting against overflow and below the cover 17 there is a connector 36 supplying technological water, and a connector 37 for returning pasteurized effluent after the operation of centrifugation, and a connector 38 for bringing the strain of microorganisms and a nutrient supply connector 39. In the side wall of the photobioreactor's tank 14 and beneath the surface of the cultivation solution there is a temperature sensor 40. In addition, the heating jacket 15 has an inlet connector 41 and a heating medium outlet connector 42. In the lower part of the side wall of the photobioreactor's tank 14 a testing valve 43 for taking samples is located. While, the cover 17 is additionally equipped with a pH sensor 44, a sterile air vent 45 and an oxygen sensor 46, and a turbidity sensor 47.

## Claims

1. Apparatus for cultivation ofphototrophic microorganisms, especially microalgae, comprising interconnected: a process water storage tank (1), a transition tank (2), an exchanger (3) formed of a pasteurization section, a recuperation section and a cooling section, an ice water aggregate (5), a heating water tank (6), a photobioreactor (7), a reproduction and heating pure cultures tank (8), a prepared nutrients heating tank (9), a carbon dioxide gas tank (10), a mixing pump (11), a centrifuge (12), a biomass storage tank (13), creating a functional installation, **characterized in that**, the photobioreactor (7) in its spacious closed tank (14) has at least one light element (19) placed inside and this element is formed of a cylindrical transparent tube (20) of ring-shaped, circular cross section and a column (22) with LED light-emitting diodes (25) embedded on its walls, that is placed inside the tube (20), wherein the tube (20) is sealed from the bottom with a lid (21) and its top edges project above the photobioreactor's tank (14) closing cover (17), while the column (22) is formed of a pass-through tube, where it's upper outlet (23) is profiled and includes a fan (24), and additionally, on the outer side of the transparent tube (20) and parallel to it's vertical axis at least two vertical collectors (26) powered by a mixing pump (11) are located, in the collectors hydraulic nozzles (27) are mounted, with it's outlets facing the outer surface of the tube (20).

2. Apparatus according to claim 1, **characterized by** the implemented light-emitting diodes LED (25) emitting light at wavelengths 450 - 470 nm - blue light, 650 - 670 nm - orange light and 700 - 740 nm - red light.

3. Apparatus according to claim 1 or 2, **characterized in that**, the inner wall of the photobioreactor's tank (14) constitutes reflective surface (30) for the light emitted.

4. Apparatus according to any one of the preceding claims, **characterized in that**, the walls of the photobioreactor's tank (14) constitute a heating jacket (15) with the outer insulation layer (16).

5. Apparatus according to any one of the preceding claims, **characterized in that**, that in the sidewall of the photobioreactor's tank (14) mixing nozzles (31) are mounted which constitute outlets of the conductors connected to the pump (11) discharge connector.

6. Apparatus according to any one of the preceding claims, **characterized in that**, that in the photobioreactor's tank (14) a turbidity sensor (47) is located.
